# EUROPÄISCHE PATENTANMELDUNG

(11) **EP 1 847 278 A1**
(43) Veröffentlichungstag der Anmeldung: **24.10.2007**
(21) Anmeldenummer: 06112659.5
(22) Anmeldetag: 13.04.2006
(51) Int. Cl.: A61L 27/06, A61L 27/58, A61L 27/30, A61C 8/00

(54) **Bio-auflösende Salzbeschichtungen von Implantaten zum Schutz vor organischen Verunreinigungen**

(71) Anmelder: Camlog Biotechnologies AG, 4053 Basel (CH)
(72) Erfinder: Denzer, Alain J., 5080, Laufenburg (CH)
(74) Vertreter: Hepp, Dieter

(57) **Zusammenfassung**

Ein Implantat, insbesondere ein Implantat für dentale Anwendungen wird wenigstens teilweise im Bereich seiner Oberfläche mit einer Schutzschicht versehen. Die Schutzschicht soll die Ablagerungen von Verschmutzungen verhindern. Die Schutzschicht ist derart ausgewählt, dass sie sich bei Kontakt mit Körperflüssigkeiten und/oder dem Knochen auflöst, so dass im wesentlichen keine Rückstände auf der Oberfläche des Implantats verbleiben.

## Beschreibung

Die Erfindung betrifft ein Implantat, eine Verpackung mit einem Implantat und ein Verfahren zum Behandeln eines Implantats mit den Merkmalen des Oberbegriffs der unabhängigen Patentansprüche. Implantate, insbesondere auch Dentalimplantate werden heute in grossen Stückzahlen eingesetzt. Solche Implantate dienen zum Einsetzen in Knochen, beispielsweise in den Kieferknochen. Solche Implantate bestehen vorzugsweise aus Titan oder aus Legierungen auf der Basis von Titan. Eine wichtige Eigenschaft der Implantate ist die so genannte Osteointegrationszeit. Darunter wird die Dauer verstanden, die vergeht, bis das Implantat ausreichend fest mit der umliegenden Knochensubstanz verbunden ist.

Der chemische Zustand der Oberfläche von Titan oder Titanbasislegierungen ist komplex. Es ist bekannt, dass die Oberfläche von Titanmetall in Luft und Wasser spontan oxidiert und es wird angenommen, dass an der Oberfläche, das heisst in der äussersten Atomschicht, eine Reaktion mit Wasser abläuft, wobei Titan-Hydroxylgruppen (TiOH), gebildet werden (Boehm H.P., 1971, Acidic and basic properties of hydroxylated metal oxide surfaces, Discussions Faraday Society, 52, 264-275).

Nach dem Modell von Baier (1972, The role of surface energy in thrombogenesis, Bull. N.Y. Acad. Med. 48, 257-272), welches für den Kontakt zwischen Blut und Biomaterial entwickelt wurde, besteht eine Korrelation zwischen Biokompatibilität, Bioadhäsion und der Oberflächenspannung des Festkörpers, bzw. des daraus berechneten Kontaktwinkels. Demzufolge verfügt eine hydrophile Oberfläche mit Kontaktwinkel 0-31° über eine sehr starke Bioadhäsion. Dagegen entsprechen Kontaktwinkel im Bereich >70° hydrophoben Oberflächen und einer hypothetischen Zone der Biokompatibilität. Die Benetzungseigenschaften bzw. der hydrophile Charakter der Implantatoberfläche kann durch Messung des Kontaktwinkels bzw. Benetzungswinkels zwischen der Flüssigkeit (Wasser) und der trockenen metallischen Substratoberfläche mittels optischer Methoden in an sich bekannter Weise bestimmt werden. Für die Bestimmung des Kontaktwinkels wird die beschichtete Oberfläche mit reinem Wasser gewaschen und in reinem Stickstoff oder Argon getrocknet. Ein Tropfen reinen Wassers wird auf die horizontal ausgerichtete Oberfläche gegeben. Durch Hinzufügen weiteren Wassers wird die Tropfenoberfläche vergrössert, was den "oberen" Kontaktwinkel ergibt, während die Entnahme von Wasser den Tropfendurchmesser im Kontakt mit der Oberfläche verkleinert, was den "unteren" Kontaktwinkel ergibt. Ein hydrophiler Charakter der Oberfläche ist gegeben, wenn der "obere" Kontaktwinkel kleiner als 50° (<50°) ist und der "untere" Kontaktwinkel weniger als 20° (<20°) beträgt.

Es ist bekannt, dass organische Verbindungen in der Luft sich direkt auf der Oberfläche von Titan- und Titanlegierungen ablagern und so die Chemie der Oberfläche verändern. Die Oberfläche wird dann hydrophob. Es sind bereits verschiedene Lösungen vorgeschlagen, wie dieses Problem gelöst werden könnte und wie dadurch die Osteointegrationszeit von Implantaten reduziert werden könnte.

Aus EP 388 576 ist ein metallisches Implantat bekannt, das eine Oberflächenrauhigkeit von mehr als 20µm aufweist. Dieser Rauhigkeit ist eine Microrauhigkeit von höchstens 2µm überlagert. Es hat sich gezeigt, dass hier wegen der von organischen Ablagerungen auf der Oberfläche die Osteointegrationszeit nachteilig beeinflusst wird.

Aus WO 00/44305 ist ein osteophiles Implantat bekannt, welches eine aufgerauhte hydroxylierte und hydrophile Oberfläche aufweist. Mindestens die hydroxylierte und hydrophile Oberfläche ist in einer gas- und flüssigkeitsdichten Umhüllung verschlossen. Die Umhüllung weist eine inerte Atmosphäre, beispielsweise aus Stickstoff und/oder teilweise aus gereinigtem Wasser auf. Ein Nachteil bei diesem Implantat ist das relativ komplizierte Verpackungsverfahren, das eine gas- und flüssigkeitsdichte Umhüllung mit inerter Atmosphäre voraussetzt.

Aus WO 03/030957 ist ein Implantat mit einer aufgerauhten hydroxylierten und hydrophilen Oberfläche bekannt, welches im hydroxylierten Zustand mit hoch energetischer ultravioletter Strahlung behandelt wird. Ein Nachteil dieser Lösung besteht im zusätzlichen Behandlungsschritt, der insbesondere durch den Chirurg durchgeführt werden soll.

Aus US 6 221 111 ist eine bioaktive Oberflächenbeschichtung für ein metallisches Implantat bekannt. Die Beschichtung besteht aus Calciumverbindungen und Metalloxiden. Das Problem von Ablagerungen aus organischem Material auf der Oberfläche des Implantats ist dadurch aber nicht gelöst.

Es ist daher eine Aufgabe der vorliegenden Erfindung, die Nachteile des Bekannten zu vermeiden, insbesondere ein Implantat, eine Verpackung und ein Behandlungsverfahren für ein Implantat zu schaffen, welches auf einfache Weise die Beeinträchtigung der biologisch aktiven Oberfläche des Implantats durch Verunreinigungen verhindert. Insbesondere soll die Erfindung ohne komplizierte Sterilverpackungen auskommen und keine weiteren aufwändigen Behandlungsschritte bedingen.

Erfindungsgemäss werden diese Aufgaben mit einem Implantat, einer Verpackung und einem Verfahren zum Behandeln eines Implantats mit den Merkmalen der unabhängigen Ansprüche gelöst.

Das erfindungsgemässe Implantat ist im besonderen ein Dentalimplantat. Die Erfindung lässt sich ebenso auch für andere Implantate anwenden. Das Implantat weist einen Implantatkörper auf, welcher zum Einsetzen und Einwachsen in einen Knochen bestimmt ist. Das Implantat ist auf der zum Einwachsen in den Knochen bestimmten Schicht wenigstens teilweise mit einer Schutzschicht versehen. Diese Schutzschicht verhindert das Ablagern von Verunreinigungen, insbesondere organischen Verbindungen auf der biologisch aktiven Oberfläche des Implantats. Erfindungsgemäss ist die Schicht derart ausgebildet, dass sie sich bei Kontakt mit Körperflüssigkeit oder bei Kontakt mit dem Knochen auflöst. Die Schicht ist so ausgestaltet, dass nach Auflösung der Schicht auf der Oberfläche im wesentlichen keine Rückstände verbleiben. Ausserdem ist die Schicht aus nach der Auflösung für den Körper unbedenklichen Bestandteilen aufgebaut.

Besonders bevorzugt ist der ganze Oberflächenbereich des Implantates, der mit dem Knochen in Kontakt kommen soll mit der Schutzschicht versehen. Es ist auch denkbar, das komplette Implantat mit einer solchen Schutzschicht zu versehen.

Gemäss einem anderen Aspekt der Erfindung ist eine Schutzschicht aus einem Salz vorgeschlagen. Bevorzugt besteht die Schicht ausschliesslich aus Salz. Es ist denkbar, die Schicht aus einem einzigen Salz oder auch aus einer Kombination von Salzen aufzubauen.

Ganz allgemein sind Schichten denkbar, welche aus in reinem Wasser gelösten Zusätzen aufgebaut sind, wobei geeignete Zusätze beispielsweise einwertige Alkalikationen, wie Na⁺ oder K⁺ oder ein Gemisch von Na⁺ und K⁺, mit entsprechenden Anionen in Form anorganischer Salze, wie zum Beispiel Natriumchlorid, Kaliumchlorid, Natrium- oder Kaliumchlorat, Natrium- oder Kaliumnitrat, Natrium- oder Kaliumphosphat oder ein Gemisch solcher Salze sind. Ebenso können auch zweiwertige Kationen in Form von wasserlöslichen anorganischen Salzen zugesetzt werden. Geeignete Kationen sind insbesondere Mg²⁺, Ca²⁺, Sr²⁺ und/oder Mn²⁺ in Form der Chloride oder deren Gemische. Geeignete Anionen sind auch Phosphat- und Phosphanatanionen, wobei darunter jeweils auch Monoorthophosphat-Anionen und Diorthophosphat-Anionen bzw. Monoorthophosphonat-Anionen und Diorthophosphonat-Anionen zu verstehen sind, in Kombination mit den genannten Kationen.

Bevorzugt weist das Salz Kationen auf, welche in der menschlichen Körperflüssigkeit vorkommen, insbesondere bevorzugt sind Kationen welche aus der Gruppe ausgewählt sind, welche aus Na⁺, K⁺, Mg²⁺, Ca²⁺ besteht.

Gemäss einem bevorzugten Ausführungsbeispiel weist die Schicht eine Dicke von wenigen Nanometern, insbesondere 1 bis 100 nm, vorzugsweise 1 bis 10 nm. Grundsätzlich ist es ausreichend, wenn die Schicht die Oberfläche abdeckt, so dass sich keine Ablagerungen darauf bilden. Bereits schon eine wenige Atomlagen dicke Schicht Ionen auf der Oberfläche verhindert, dass organische Verbindungen sich direkt auf der Ti-Oberfläche ablagern. Obwohl sich die organischen Verbindungen dann auf der Salzschicht ablagern können, bleibt die Oberfläche durch den Schutz der Ionen insgesamt hydrophil und biologisch wirksam.

TiOH (Titanhydroxyl) in der äussersten Atomlage der Oberfläche entsteht durch Anlagerung von H₂O. Je nach Säurewert ist die Oberfläche negativ (TiO⁻) oder positiv (TiOH₂⁺) geladen und bestimmt daher, welches Ion in der ersten atomaren Schicht adsorbiert wird. Der Isoelektrische Punkt von Titan liegt im Bereich pH6-6.5. Demzufolge werden Anionen adsorbiert, wenn der pH-Wert unter 6 liegt und Kationen, wenn der pH-Wert über 6.5 liegt.

Die biologische Wirksamkeit der mit einer Schutzschicht, insbesondere Ionen beschichteten Oberfläche lässt sich damit erklären, dass nach Implantation das Wasser aus den Körperflüssigkeiten durch die Schicht, insbesondere Ionen auf der Implantatoberfläche angezogen und gebunden wird. Damit ist der Weg frei für die Adsorption von diversen Ionen aus dem Blut, der Interaktion mit Biomolekülen (Proteine, Lipide, Lipoproteine und Peptide) und schliesslich der Anlagerung von Knochenzellen. Bei rauen hydrophoben Oberflächen bilden sich dagegen Luftblasen in den Kavitäten und verhindern damit den direkten Kontakt der Köperflüssigkeiten mit der Oberfläche. Dieses Phänomen führt zu einer Verzögerung der Adsorption von Biomolekülen aus den Körperflüssigkeiten und infolgedessen zu einer langsameren knöchernen Einheilung des Implantats.

Gemäss einem bevorzugten Ausführungsbeispiel weist der Implantatkörper eine Oberfläche mit einer Makrorauhigkeit auf. Die Makrorauhigkeit kann typischerweise durch Sandstrahlen mit einem Korn der mittleren Korngrösse von 0,1mm bis 0,5mm erzielt werden. Typische solche Strukturen sind beispielsweise aus EP 388 576 und aus im Markt erhältlichen Implantaten bekannt.

Besonders bevorzugt ist es, wenn die Oberfläche zusätzlich mit einer Mikrorauhigkeit versehen ist. Die Erzeugung einer Mikrorauhigkeit der Oberfläche erfolgt vorzugsweise mit einer anorganischen Säure oder einem Gemisch anorganischer Säuren, vorzugsweise mit Fluorwasserstoffsäure, Chlorwasserstoffsäure, Schwefelsäure, Salpetersäure oder einem Gemisch solcher Säuren.

Beispielsweise kann eine Behandlung mit einem wässrigen Chlorwasser Stoffsäure/Schwefelsäure-Gemisch mit einem Verhältnis HCl: H₂SO₄: H₂O: von 2:1:1 bei >80°C und während 1 bis 10 min. erfolgen. Eine derartige Behandlung ist bereits aus EP 388 576 bekannt.

Ebenso bevorzugt ist es, wenn die Oberfläche zusätzlich mit einer Nanorauhigkeit versehen ist. Die Erzeugung einer Nanorauhigkeit der Oberfläche erfolgt vorzugsweise mit einer alkalischen Lösung, insbesondere eines Alkalihydroxid, vorzugsweise mit Natriumhydroxid oder Kaliumhydroxid.

Bevorzugt besteht die Schutzschicht ausschliesslich aus einem einzigen Salz, welches direkt auf die gereinigte, insbesondere von organischen Verbindungen befreite Oberfläche des Implantats aufgetragen ist.

Bevorzugt besteht das Implantat aus Titan oder aus Titanlegierungen. Es wären aber auch andere Materialien grundsätzlich denkbar.

Die Erfindung verkürzt die Osteointegrationszeit von Implantaten mit einer Schicht aus wasserlöslichen Salzkristallen auf der chemisch sauberen, rauen Implantatoberfläche. Vor allem in den ersten 6 Wochen der knöchernen Wundheilung sind die besseren Ergebnisse zu beobachten.

Ein weiterer Aspekt der Erfindung betrifft eine Verpackung mit einem vorstehend beschriebenen Implantat. Das Implantat wird in einem Raum der Verpackung aufbewahrt. Die Schicht bildet eine Konservierungsschicht zum Verhindern von Ablagerungen auf der Oberfläche des Implantates. Daher ist es nicht erforderlich, die Verpackung, gas- und/oder flüssigkeitsdicht auszubilden und wie im Stand der Technik mit einer inerten Atmosphäre zu versetzen. Die Anforderungen an das Herstellverfahren sind daher geringer.

Ein weiterer Aspekt der vorliegenden Erfindung betrifft ein Verfahren zum Behandeln eines Implantates. Dazu wird die Oberfläche des Implantates nötigenfalls gereinigt. Dabei werden insbesondere organische Ablagerungen entfernt. Die auf diese Weise gereinigte Oberfläche wird anschliessend mit einer Schutzschicht versehen, insbesondere einer Schutzschicht aus Salz.

Besonders einfach lässt sich die Schutzschicht auf das Implantat aufbringen, in dem das Implantat in eine Salzlösung eingetaucht und anschliessend getrocknet wird. Typischerweise kann eine 0,01M bis 1M Lösung, insbesondere eine 0,1M NaCl Lösung verwendet werden. Dazu werden Salze in reines Wasser gegeben, so dass nach dem Trocknen die auf der Implantatoberfläche abgelagerte Schicht ausschliesslich aus dem oder den gewünschten Stoffen, insbesondere Salzen bestehen.

Damit bei der Trocknung keine schädlichen Rückstände auf der Oberfläche abgelagert werden, erfolgt die Trocknung insbesondere mit einem inerten Material, beispielsweise mit Stickstoff. Als Bestandteile der Schutzschicht kommen insbesondere die vorstehend beschriebenen Kationen und Anionen zur Anwendung.

Durch Eintauchen des Implantats in die beschriebene Lösung und durch anschliessendes Trocknen wird typischerweise eine Schicht von wenigen Nanometern, insbesondere 1 bis 100 nm, vorzugsweise 1 bis 10 nm erzeugt.

Noch ein weiterer Aspekt der Erfindung betrifft die Verwendung einer Salzschicht auf der biologisch aktiven Oberfläche eines Implantates zum Schutz wenigstens eines Teils der Oberfläche des Implantates gegen Verunreinigungen.

Die Erfindung wird im folgenden an Hand von Ausführungsbeispielen näher erläutert.

### Beispiele:

Die Zahnimplantate werden zerspannend durch Drehen und Fräsen eines zylindrischen Rohlings in an sich bekannter Weise erhalten. Die Oberfläche, welche direkt mit dem Knochen in Kontakt kommt, wird mit einer Makrorauhigkkeit versehen, indem diese mit einem Korn der mittleren Korngrösse 0.1-0.5mm sandgestrahlt wird.

Anschliessend wird die aufgeraute Oberfläche (Makrorauhigkeit) mit einem wässerigen Chlorwasserstoffsäure/Schwefelsäuregemisch mit einem Verhältnis HCl:H₂SO₄:H₂O von 2:1:1 bei einer Temperatur von >80°C während 1-10 Minuten behandelt, um eine definierte, die Makrorauhigkeit überlagernde Mikrorauhigkeit zu erhalten.

### Beispiel 1:

Das so geformte Implantat wird sofort mit einer reinen 0.15M NaCl-Lösung neutralisiert, aus der Lösung entfernt und mit Stickstoff getrocknet. Das derart mit einer Schicht versehene Implantat wird anschliessend in einer Verpackung aufbewahrt.

### Beispiel 2:

Das so geformte Implantat wird in reinem Wasser neutralisiert und die aufgerauhte Oberfläche (Makrorauhigkeit/Mikrorauhigkeit) mit 3M KOH bei einer Temperatur von >60°C während 10-30 Minuten behandelt, um eine definierte, die Mikro-/Makrorauhigkeit überlagernde Nanorauhigkeit zu erhalten. Anschliessend wird das strukturierte Implantat sofort mit einer reinen 0.15M NaCl-Lösung neutralisiert, aus der Lösung entfernt und mit Stickstoff getrocknet. Das derart mit einer Salzschicht versehene Implantat wird anschliessend in einer Verpackung aufbewahrt.

### Beipiel 3:

Das so geformte Implantat wird in reinem Wasser neutralisiert und an Luft bei 80-110°C getrocknet. Danach wird die Oberfläche mit einer UV/Ozon-Behandlung gereinigt und sofort in eine reine 0.15M NaCl-Lösung eingetaucht, bevor sie aus der Lösung entfernt und mit Stickstoff getrocknet wird. Bei diesem zweiten Ausführungsbeispiel können allenfalls nach der Säurebehandlung wieder abgelagerte Verunreinigungen entfernt werden. Das auf diese Weise geschützte Implantat wird in eine Verpackung gegeben und darin aufbewahrt.

### Beispiel 4:

Das so geformte Implantat wird in reinem Wasser neutralisiert und an Luft bei 80-110°C getrocknet. Danach wird die Oberfläche mit einer Plasma-Behandlung gereinigt und sofort in eine reine 0.15M NaCl-Lösung eingetaucht, bevor sie aus der Lösung entfernt und mit Stickstoff getrocknet wird.

Die Beschichtung der Oberfläche mit Ionen kann durch XPS (x-ray photoelectron spectroscopy) und AES (Auger electron spectroscopy) analysiert werden. Eine gute Ionenbeschichtung ist, wie vorstehend dargelegt, nur wenige nm dick und weist 1-50 Atomprozent positiv und negativ geladene Ionen auf.

Resultate von XPS-Messung bei Verwendung von:
0.01M NaCl : 5% Na⁺, 2% Cl⁻
0.1M NaCl : 20% Na⁺, 12% Cl⁻
1M NaCl : 40% Na⁺, 25% Cl⁻

Wie fest das Implantat im Knochen verankert ist, lässt sich mechanisch bestimmen, nämlich durch Messung der Kraft, sei es als Zug, Druck, Scherung oder Drehmoment, welche nötig sind, um das im Knochen verankerte Implantat aus seiner Verankerung herauszuziehen oder herauszudrehen. Messungen haben gezeigt, dass sich Titanimplantate mit glatter Oberflächenstruktur nur ungenügend im Knochen verankern, während Implantate mit aufgerauter Oberfläche einen bezüglich der Zugfestigkeit merklich verbesserten Knochen-Implantat-Verbund ergeben. Es hat sich gezeigt, dass Implantate mit einer Beschichtung aus Ionen besser osteointegrieren als Implantate ohne Salzbeschichtung.

Eine weitere Möglichkeit, die verbesserte Osteointegration festzustellen, ist durch Messung des bone implant contact. Dazu werden histologische Schnitte des im Knochen verankerten Implantats unter dem Lichtmikroskop systematisch ausgewertet. Es hat dich auch hier gezeigt, dass Implantate mit einer Beschichtung aus Ionen besser osteointegrieren als Implantate ohne Salzbeschichtung.

## Patentansprüche

1. Implantat, insbesondere Dentalimplantat,
mit einem Implantatkörper, welcher zum Einsetzen in einen Knochen bestimmt ist, **dadurch gekennzeichnet, dass** wenigstens der Implantatkörper mindestens teilweise mit einer Schutzschicht versehen ist, welche sich bei Kontakt mit Körperflüssigkeit und/oder den Knochen auflöst.

2. Implantat insbesondere nach Anspruch 1, mit einem Implantatkörper, welcher zum Einsetzen in einen Knochen bestimmt ist,
**dadurch gekennzeichnet, dass** wenigstens der Implantatkörper mindestens teilweise mit einer Schutzschicht versehen ist,
wobei die Schutzschicht aus Salz besteht.

3. Implantat nach Anspruch 2, **dadurch gekennzeichnet, dass** das Salz aus Kationen besteht, welche in der menschlichen Körperflüssigkeit vorkommen, insbesondere aus der Gruppe bestehend aus Na⁺, K⁺, Mg²⁺, Ca²⁺ ausgewählte Kationen.

4. Implantat nach einem der Ansprüche 2 oder 3, **dadurch gekennzeichnet, dass** das Anion des Salzes Cl⁻ oder Phosphat ist.

5. Implantat nach einem der Ansprüche 1 bis 4, **dadurch gekennzeichnet, dass** die Schicht eine Dichte von wenigen Nanometern, insbesondere 1 bis 100 nm, vorzugsweise 1 bis 10 nm aufweist.

6. Implantat nach einem der Ansprüche 1 bis 4, **dadurch gekennzeichnet, dass** der Implantatkörper eine Oberfläche mit einer Makrorauhigkeit aufweist, insbesondere eine Makrorauhigkeit welche durch Sandstrahlen mit einem Korn der mittleren Korngrösse von 0,1mm bis 0,5mm erzielbar ist.

7. Implantat nach einem der Ansprüche 1 bis 6, **dadurch gekennzeichnet, dass** der Implantatkörper eine Oberfläche mit einer Mikrorauhigkeit aufweist, insbesondere eine Mikrorauhigkeit welche durch Säurebehandlung erzielbar ist, insbesondere durch Behandlung mit einem wässerigen Chlorwasserstoffsäure/Schwefelsäuregemisch mit einem Verhältnis HCl:H₂SO₄:H₂O von 2:1:1 bei >80° Celsius während 1 bis 10 min.

8. Implantat nach einem der Ansprüche 1 bis 7, **dadurch gekennzeichnet, dass** es eine Oberfläche mit einer Nanorauhigkeit aufweist, insbesondere eine Nanorauhigkeit, welche durch alkalisches Ätzen erzielbar ist, insbesondere durch Behandlung mit einer Lösung eines Alkalihydroxids, vorzugsweise Natriumhydroxid oder Kaliumhydroxid, insbesondere bei einer Konzentration im Bereich von 1-5M, vorzugsweise bei >55°C, insbesondere während 10-30 Minuten.

9. Implantat nach einem der Ansprüche 2 bis 8, **dadurch gekennzeichnet, dass** die Schicht ausschliesslich aus einem einzigen Salz besteht, welches direkt auf eine vorgereinigte Oberfläche, insbesondere von organischen Verbindungen befreite Oberfläche des Implantats aufgetragen ist.

10. Implantat nach einem der Ansprüche 1 bis 8, **dadurch gekennzeichnet, dass** der Implantatkörper aus Titan oder einer Titanlegierung besteht.

11. Verpackung mit einem Implantat nach einem der Ansprüche 1 bis 10, wobei das Implantat in einem Raum der Verpackung gehalten ist und wobei die Schutzschicht, insbesondere die Salzschicht eine Schicht zum Verhindern von Ablagerungen auf der Oberfläche des Implantates bildet.

12. Verfahren zum Behandeln eines Implantats, **dadurch gekennzeichnet, dass** die Oberfläche des Implantats wenigstens teilweise mit einer Schutzschicht, insbesondere einer Schicht aus Salz versehen wird, welche sich bei Kontakt mit Körperflüssigkeit auflöst.

13. Verfahren nach Anspruch 12, **dadurch gekennzeichnet, dass** die Oberfläche des Implantats vor dem Aufbringen der Schutzschicht gereinigt, insbesondere von organischen Ablagerungen befreit wird.

14. Verfahren nach einem der Ansprüche 12 oder 13, **dadurch gekennzeichnet, dass** die Schicht durch Eintauchen des Implantates in eine Salzlösung und durch anschliessendes Trocknen aufgebracht wird.

15. Verfahren nach Anspruch 14, **dadurch gekennzeichnet, dass** das Implantat in eine 0,01 M - 1M NaCl-Lösung, insbesondere eine 0,15M NaCl Lösung eingetaucht wird.

16. Verfahren nach einem der Ansprüche 14 oder 15, **dadurch gekennzeichnet, dass** die Oberfläche nach dem Entfernen aus der Lösung mit einem inerten Material, insbesondere mit Stickstoff getrocknet wird.

17. Verfahren nach einem der Ansprüche 15 oder 16, **dadurch gekennzeichnet, dass** die Lösung Kationen aufweist, welche in der menschlichen Körperflüssigkeit vorkommen, insbesondere Kationen, welche aus der Gruppe bestehend aus Na⁺, K⁺, Mg²⁺ und Ca²⁺ ausgewählt sind.

18. Verfahren nach einem der Ansprüche 14 bis 17, **dadurch gekennzeichnet, dass** das Anion in der Lösung Cl⁻ oder Phosphat ist.

19. Verfahren nach einem der Ansprüche 12 bis 18, **dadurch gekennzeichnet, dass** die Schutzschicht in einer Dicke von wenigen Nanometern, insbesondere 1 bis 100 nm, vorzugsweise 1 bis 10 nm aufgetragen wird.

20. Verwendung einer in Kontakt mit Körperflüssigkeit löslichen Schicht, insbesondere einer Salzschicht, auf der biologischen Oberfläche eines Implantats zum Schutz wenigstens eines Teils der Oberfläche gegen Verunreinigungen.
